# EUROPEAN PATENT SPECIFICATION

(11) **EP 0 408 734 B1**
(45) Date of publication and mention of the grant of the patent: **02.02.1994**
(21) Application number: 90903240.1
(22) Date of filing: 05.02.1990
(51) Int. Cl.: C12N 9/70

(54) **A METHOD FOR CLEANSING STREPTOKINASES**
VERFAHREN ZUR REINIGUNG VON STREPTOKINASEN
PROCEDE DE NETTOYAGE DE STREPTOKINASES

(30) Priority: 09.02.1989 SE 8900444
(43) Date of publication of application: 23.01.1991
(73) Proprietor: Pharmacia & Upjohn Aktiebolag, 112 87 Stockholm (SE)
(72) Inventor: EINARSSON, Monica, S-752 52 Uppsala (SE); GELLERBRING, Ann-Kristin, S-113 38 Stockholm (SE); LARSSON, Torbjörn, S-172 47 Sundbyberg (SE)
(74) Representative: Tannerfeldt, Sigrid Agneta
(86) International application number: SE9000073
(87) International publication number: WO9009439

(56) References cited:
- US-A- 3 107 203
- US-A- 3 917 527

## Description

The present invention relates to the cleansing of streptokinases and derivatives and fragments thereof.

Clotting of the blood in an artery or a vein of the human body is one of the most common causes of mortality and invalidity in the industrialized nations. Those diseases caused by clotting of the blood, embolism or thrombosis, include cardiac infarct, coronary thrombosis, strokes, pulmonary embolism, deep venous thrombosis and peripheral arterial occlusions. The medical treatment available is either one of preventing further development of the clot or embolus and of reducing the risk of further blood clots developing, or one of dissolving the blood clot(s). In the last thirty years, two preparations, streptokinase and urokinase, have been used to promote dissolution of blood clots. These preparations function to activate the blood-clot dissolution system of the body and to convert the inactive proenzyme plasminogen to the proteolytic active enzyme plasmin, which is capable of breaking down the fibrin contained in the blood clot or embolus and therewith dissolve the clot.

Streptokinase is a protein which is produced under given conditions from various strains of streptococci (e.g. β hemolytic streptococci, Lancefield Group C). By fermenting these streptococci on a large scale, it is possible to obtain sufficient quantities of streptokinase-containing broth from which active streptokinase can be recovered for extensive clinical use, subsequent to thoroughly cleansing the broth.

Streptokinase has been used in the treatment of deep venous thrombosis (blood clots in arms and legs) since the 1960s, by intravenous infusion over a long period of time (several days) (see Drugs 5, 357-455 (1973) Streptokinase. A Review). At the beginning of the 1980s, it was shown, inter alia, by coronary angiography, that one or more blood clots are present in the coronary vessels in 90% of the cases of acute coronary thrombosis (heart attacks). In the majority of cases, it was possible to dissolve these clots by treatment with streptokinase. This treatment was effected initially by intracoronary administration and later by intravenous infusion, in high dosages over a short period of time (30-60 minutes). These new experiences have resulted in a considerable increase in the use of streptokinase in recent years.

The clinical use of streptokinase in connection with acute coronary thrombosis has enabled the mortality rate to be lowered by up to 50% (see The Lancet 13, August 1988, pages 349-360). The treatment is not totally problem-free, however. Firstly, there is a slight risk that internal bleeding will occur, which is only to be expected when treatment is effected with different thrombolysis agents. Secondly, the treatment can result in low-grade pyrexia and in low-frequency minor allergic reactions, both of which effects are of a temporary nature. Since streptokinase is taken from a bacterium, it can itself give rise to fever and allergic reactions. Any contaminated components of the preparations can also cause such fever and allergic reactions, at least theoretically.

The process by means of which streptokinase is cleansed is of the highest importance. Firstly, the process shall ensure that the streptokinase is as pure as possible, so as to reduce the risk of allergical secondary effects, and secondly, the process shall be capable of producing a clinically active preparation in high yields. Furthermore, the process will preferably enable streptokinase to be cleansed readily and quickly, so that production costs can be reduced.

The processes at present used for the cleansing of streptokinase include such conventional aids and methods as surfactants, salt precipitations, absorption chromatography, ion-exchange chromatography and gel-filtration. (See, for instance, Biochemica et Biophysica Acta, 1979, 19-29, Einarsson et al and US 3 098 015). These cleansing processes are time-consuming and costly, however, because of the many separation stages required, and the yields are relatively low. Furthermore, the various chromatographic cleansing stages involved, to a large extent, the use of relatively non-specific matrices which possess poor flow characteristics (e.g. Kaolin). The majority of the cleansing processes employed today were developed thirty years ago and no longer fulfill present-day high requirements.

In conjunction with investigations into the properties of streptokinase, it was found, surprisingly, that streptokinase will bind to hydrophobic ligands coupled to different gel-matrices. Elution can then be effected with a solution whose ion-strength, pH, hydrophility and/or temperature differ from corresponding parameters of the solution used to bind the streptokinase to the gel.

More than 90% of the streptokinase will bind to these ligands in the presence of salt of high concentration, whereas the major part of contaminated proteins will not bind to said ligands and can readily be washed-out. The streptokinase can then be eluted from the matrix, by lowering the salt concentration in the eluting agent.

Accordingly, the present invention relates to a method of cleansing a streptokinase and derivatives and fragments thereof, and is characterized by causing the streptokinase in solution to bind to a gel consisting of a matrix whith hydrophobic ligands containing linear, branched or cyclic hydrocarbon groups having more than four carbon atoms, and subsequently eluting from the gel the streptokinase bound to said ligands.

The inventive method can be employed for cleansing derivatives of streptokinase, and also for cleansing fragments of streptokinase, where the streptokinase molecule has been partially degraded. The term "streptokinase", as used in the present description and in the following claims, is also meant to include such derivatives and fragments.

US-A-3 917 527 describes the separation and purification of various biomolemules by selectively and reversibly binding them to an adsorbent in a chromatographic column, said adsorbent comprising a solid matrix support having attached thereto hydrocarbon arms. This reference is directed to the basic idea, which encompasses a very great number of carriers, ligands and products to be purified. The expression "biomolecules" covers an almost infinite number of compounds. In most of the examples of the reference, it is shown that a longer carbon chain gives an inferior result in the purification. This speaks against the idea of the present invention, according to which the ligand groups must have more than four carbon atoms. As far as the applicant is aware, the process described in the reference has not found any use in industrial production.

It is known that hydrophobic gel-matrices are able to bind macromolecules, and that this property has been utilized to separate or extract viruses, for instance. (See J. Virol Methods, 1981, 3:213-38, Einarsson et al and US 4 138 287), or for cleansing proteins on a laboratory scale. Such processes are difficult to control, particularly when attempting to carry out such processes on a larger, industrial scale. Such attempts will often result in the denaturization and destruction of macromolecules, or render it difficult to recover such molecules in a pure form with retained high activity and retained high yields. In accordance with the present invention, however, it has been found, surprisingly, that a hydrophobic gel is highly suitable for the cleansing of streptokinases.

The matrix material may comprise one of a number of known materials. Particularly preferred are those hydrophilic materials which are known in connection with gel filtration, such as agarose gels (e.g. Sepharose® from AB Pharmacia), dextran gels (e.g. Sephadex® from AB Pharmacia), cellulosic gel-matrices and acrylamide gel-matrices.

The hydrophobic ligands bound to the hydrophilic matrix may be of various kinds. The ligands will primarily consist of hydrocarbon groups of linear, branched or cyclic type, and will contain more than four carbon atoms. In the case of groups which contain only four or fewer carbon atoms, the hydrophobic properties will not be sufficiently pronounced to achieve good binding of the enzyme to the gel. The ligands become successively more hydrophobic with increasing numbers of carbon atoms, wherewith the bond between the enzyme and the ligands becomes successively stronger. An excessively strong bond can result in elution difficulties, requiring the use of special remedial measures, and the yield will decrease. When the ligands are excessively hydrophobic, the gel will become "greasy", and can no longer be wetted sufficiently by the water phase. Furthermore, the density of the gel will also decrease, causing the gel to flocculate and finally float in water, and difficulties will arise in producing a standard column.

Consequently, this places an upper, practical limit on the size of the hydrophobic groups, although this limit is not sharply defined and a gradual transition exists.

The proportion of hydrophobic groups on the matrix can vary within wide limits. It is in this regard that the total hydrophobic properties of the matrix with the ligands have significance. The size of the groups bound to the matrix is also influential in this regard, such that a large number of ligands having few carbon atoms is required to provide the same hydrophobic properties as a smaller number of ligands having a larger number of carbon atoms (see Experimentia 32, 456 (1976)).

Aliphatic hydrocarbon groups which can be bound to the matrix material may comprise straight-chain or branched pentyl, hexyl, heptyl, octyl, nonyl or decyl groups. Decyl groups are able to bind the enzyme so firmly as to require the use of special measures during the elution process, and in the case the upper practical limit mentioned above is approached with regard to the size of the ligands.

Those cyclic hydrocarbon groups which can be bound to the matrix material may comprise alicyclic, aromatic or araliphatic groups, such as phenyl, benzyl, cyclohexyl or the like. Hydrocarbon-substituted cyclic groups, such as tolyl, xylyl and methyl cyclohexyl can also be used.

The matrix materials having hydrophobic ligands bound thereto can be prepared by means of methods known from the literature. (Carbohydrate Res. 58, 249-51, 1977, Larm et al and Nature (1967), 214, 1302-04, Axén et al).

The streptokinase can be eluted from the hydrophobic ligands on the matrix in accordance with various principles, as disclosed in the aforegoing. For instance, the streptokinase can be supplied to the gel in the form of a solution which contains a high concentration of salt, and elution can be subsequently effected with a solution of lower salt-content and therewith lower ion strength. The upper limit of the salt content of the supplied solution is determined by the saturation content, although a more practical upper limit is that limit above which the streptokinase will precipitate from the solution. This limit can be readily established by one skilled in this art, by simple routine experiment. The lower limit of the salt content is not critical and in practice can be set at zero, that is pure water. Normally, however, the elution process is effected with a buffer solution of appropriate ion strength. Elution can also be effected with salt contents which change stepwise or continuously, i.e. a gradient.

The salts used are primarily chlorides, sulphates or phosphates of alkali metal, such as sodium or potassium, or of ammonium. These salts, however, are not the only salts that can be used, and in principle there can be used any salt whatsoever which will not have any detrimental effect on the materials or the process.

A further elution principle involves changing the pH of the eluting solution with respect to the solution used to bind the streptokinase to the matrix. In such cases, the elution process will normally be carried out at a higher pH than that used in binding the streptokinase. The ingoing solution of streptokinase will thus have a pH whose lower limit is determined by the pH at which the streptokinase is denatured or irreversibly inactivated in some way. The upper pH-limit in the elution process is determined by the pH at which the streptokinase or gel will be disadvantageously affected. The elution process is preferably carried out at an alkaline pH. The pH limits are not critical, however, and can be established by the skilled person with the aid of simple routine experiments.

The desired pH-values of the solutions used are obtained preferably with the aid of different buffer solutions capable of being prepared readily by one skilled in this art on the basis of information contained in the literature, once having established the pH value desired.

A further elution principle involves the use of an eluting solution having different hydrophilic properties with respect to the solution used to bind the streptokinase to the matrix. In this respect, the eluting solution will normally be more hydrophilic than the ingoing streptokinase-solution. This can be achieved with an eluting solution which consists of an aqueous solvent-solution. Such solvents will primarily comprise low alkanols, such as methanol, ethanol, propanol, isopropanol and butanol, although other solvents can also be used, such as ethylene glycol, glycerole and acetone, and also mixtures of such solvents. The solvent most preferred is ethanol.

The solvent content of the eluting solution can be selected within wide limits. One criterion for determining the upper limit of the solvent content is that the streptokinase and/or the gel must not be affected deleteriously. For instance, high proportions of ethanol in the solution can cause the streptokinase to precipitate or to be denatured. The criterion determining the bottom limit of solvent in the eluting solution is that a sufficiently large difference in hydrophility is obtained in respect to the input solution of streptokinase. The input solution of streptokinase can also contain organic solvent, although it is imperative that the difference in hydrophility with respect to the eluting solution is sufficient to enable satisfactory separation to be achieved.

Finally, the eluting process can be effected by bringing the eluting solution to a temperature different to the temperature of the streptokinase input solution. The binding strength changes with temperature, and the bond between streptokinase and hydrophobic ligand will be stronger at higher temperatures. Consequently, the eluting solution shall have a lower temperature than the input solution. The effect of a temperature change will be comparatively small within the temperature range capable of being applied in practice in streptokinase-cleansing processes, and solely a change in the temperature of the eluting solution will normally be insufficient to achieve satisfactory separation. It can, however, constitute a contributing factor when eluting in accordance with one of the aforesaid principles.

The different eluting methods described in the aforegoing can also be combined. For instance, an eluting solution having a low ion strength can also be buffered to a high pH-value and/or may be admixed with an organic solvent. This will enable an amplified separation effect to be achieved. When a combination of eluting methods is used, it is important that no deleterious reactions occur, such as precipitations or other harmful influences on the streptokinase or gel.

The actual separation process is carried out in a known manner. The gel matrix incorporating the hydrophobic ligands is preferably arranged in a conventional column. The enzyme is bound to the gel, by passing a solution through the column, wherewith the major part of the contaminants present will not bind to the ligands. The column is then suitably rinsed with a solution which is buffered to an appropriate pH and ion strength so that no elution takes place, but simply that the contaminants are further washed-out. Elution is then effected in accordance with one of the previously described principle, either one of said principles or a combination thereof. The elution process can be carried out stepwise or by means of a gradient, in a known manner.

If so desired, the cleansed streptokinase can be subjected to a further, conventional cleansing stage, such as gel filtration or ion-exchange chromatography. This will ensure that a high-grade streptokinase of sufficiently highly purity will always be obtained for clinical use.

However, the hydrophobic chromatography proposed in accordance with the present invention will provide a streptokinase of sufficient purity, so as to obviate the need for further cleansing stages.

The inventive method provides a substantially higher yield and purer product than the earlier known cleansing techniques.

It is found that the inventive method provides a streptokinase-yield of about 95%, as compared with a yield of about 45% when practicing earlier known methods. The degree of purity achieved is also found to be twice that achieved with earlier known techniques. The number of separation stages required when practicing the present invention is also fewer than those required when practicing known techniques, and consequently the inventive process is less expensive and takes less time to effect than said known techniques, since the degree of purity achieved by the hydrophobic chromatography is sufficiently high to obviate the need of further purification stages, or requires the use of fewer stages.

A further advantage afforded by the inventive method resides in the improvement of the working environment. The matrix material mostly used, i.e. kaolin, has often caused allergic reactions when separating the material from the enzyme product on an industrial scale. These reactions may have been caused in sensitive users by kaolin dust with enzyme residues adhering thereto. This drawback is eliminated totally by the separation method proposed in accordance with the present invention.

The invention will now be further illustrated with reference to the following Examples:

### Example 1

β-hemolytic streptococci were fermented in 5 litre fermentation tanks with a nutrient at pH 7.1. Fermentation was continued for seven hours at +35°C, whereafter the temperature was lowered to +10°C and the pH raised to 7.7. Cells and cell residues were then removed by centrifuging at 2,100 g. The solution was sterile-filtered and a concentrated solution of ammonium sulphate was added until a 21% degree of saturation was obtained. The streptokinase-content of the solution was in excess of 5,000 international units/ml.

About 10 litres of this solution was then passed through a column containing about 250 ml octyl-Sepharose®-gel which had previously been equalized with ammonium sulphate (21% saturation degree) containing 0.02 M phosphate buffer, pH 7.4. All streptokinase activity was bound to the column, whereas more than 90% of the contaminants passed straight through. Subsequent to washing the column with an equalizing buffer, the streptokinase was eluted with 0.02 M phosphate buffer, pH 7.4. The yield was 82% of the activity applied to the column. The specific activity was increased from 3 international units per microgram of nitrogen to 210 international units per microgram of nitrogen. Subsequent to two further cleansing stages, gel filtration on Sephadex®G-150 and ion-exchange chromatography on DEAE-Sepharose®, there was obtained a streptokinase having a degree of purity higher than 90% and a specific activity of 710 international units per microgram of nitrogen.

The octyl-Sepharose®-gel was prepared by coupling octylamine to Sepharose® with bromoxidation and reductive aminization (for further details, see Larm and Scholander, Carbohydrate Res. 58, 249-251 (1977)). The substitution degree was 3.4 mol-percent ligand per mol galactose. Other coupling methods were used, such as the cyanogen-bromide method (see Nature, )1967), 214, 1302-04, Axén et al).

### Example 2

The method was carried out in a manner similar to the method recited in Example 1, with the exception that elution from the octyl-Sepharose®-column was effected with a reverse salt gradient from 21% saturation degree to 0 in 0.02 M phosphate buffer, pH 7.4. The yield in this stage was 78% and after the last cleansing stage, there was obtained, in total, a specific activity of 720 international units/microgram of nitrogen.

### Example 3

β-hemolytic streptococci were fermented in 5-litre fermentation tanks with a nutrient, at pH 7.1. Subsequent to seven hours fermentation at +35°C, the temperature was lowered to +10°C and the pH raised to 7.7. Cells and cell residues were then removed by centrifugation, at 2,100 g. The solution was filtered-off and a concentrated solution of ammonium sulphate was added until an 18% degree of saturation was obtained. About 30 litres of the solution was passed through a column containing 0.6 litre phenyl-Sepharose® solid flow (Pharmacia), which was equalized with 0.02 M phosphate buffer, containing ammonium sulphate, at pH 7.4 until an 18% degree of saturation was reached. All of the streptokinase activity was bound, whereas the major part of the contaminants passed straight through the column. The column was then washed and the streptokinase subsequently eluted with 0.02 M phosphate buffer, pH 7.4. The yield was 85% of the activity applied to the column. The specific activity increased from 3 international units per microgram nitrogen to 201 international units. Subsequent to a further two cleansing stages, the streptokinase obtained had a degree of purty in excess of 90%.

### Example 4

Heptyl-agarose-gel was prepared by coupling heptyl amine to Sepharose® with the aid of bromooxidation and reductive amination (for more details, see Larm and Scholander, Carbohydrate Res. 58, 249-251 (1977)). The degree of substitution was 4.2 mol-percent ligand per mol galactose.

β-hemolytic streptococci were fermented in 5-litre fermentation tanks in the presence of a nutrient, at pH 7.1. Subsequent to seven hours fermentation at +35°C, the temperature was lowered to +10°C and the pH was raised to 7.7. Cells and cell residues were then removed by centrifugation at 2,100 g. The solution was filtered and a concentrated solution of ammonium sulphate was added until a saturation degree of 21% was reached. 150 ml solution was passed through a column containing 15 ml heptyl-agarose-gel which had previously been equalized with ammonium sulphate (21% degre of saturation) containing 0.02 M phosphate buffer pH 7.4. All the streptokinase activity was bound to the column, whereas more than 90% of the contaminants passed straight through. Subsequent to washing the column with equalizing buffer, the streptokinase was eluted with 0.02 M phosphate buffer, pH 7.4. The yield equalled 84% of the activity applied to the column.

### Example 5

Nonyl-agarose-gel was prepared in the manner described in Example 4.

The method was carried out in the same manner as in Example 4, but with the exception that a column comprising nonylamine-agrose-gel was used. As similar to Example 4, all of the streptokinase was bonded to the column, whereas 88% of the contaminants passed straight through. The yield equalled 80% of the streptokinase activity applied to the column.

### Example 6

Decylamine-agarose-gel was prepared in a manner similar to that described in Example 4.

The method was carried out in the same manner as the method in Example 4, with the exception that a column comprising decyl-agarose-gel was used. All of the streptokinase activity bonded to the column, whereas 87% of the contaminants passed straight through. Subsequent to washing the column, the streptokinase was eluted with 0.06 M phosphate buffer, pH 11.5. The elute was neutralized immediately, so as to retain protein activity. The yield was 75% of the activity applied to the column.

## Claims

1. A method for cleansing streptokinases and derivatives and fragments thereof, **characterized** by causing a streptokinase in solution to bind to a gel comprising a matrix with hydrophobic ligands containing linear, branched or cyclic hydrocarbon groups having more than four carbon atoms, and subsequently eluting from the gel streptokinase which has bonded to the ligands.

2. A method according to Claim 1, **characterized** in that the streptokinase is bound to the ligands by bringing the streptokinase in solution in a salt solution having a salt content beneath the saturation content into contact with the gel.

3. A method according to Claim 2, **characterized** in that the salt is one or more sulphates, chlorides and/or phosphates of alkali metal or ammonium.

4. A method according to any one of Claims 1-3, **characterized** in that the streptokinase is eluted by supplying a salt solution of lower ion strength than the ion-strength of the solution used to bind the streptokinase to the gel.

5. A method according to any one of Claims 1-3, **characterized** in that the streptokinase is eluted by supplying a solution whose pH differs from the pH of the solution used to bind streptokinase to the gel.

6. A method according to any one of Claims 1-3, **characterized** in that the streptokinase is eluted by supplying a solution which has greater hydrophilicity than the solution used in binding the streptokinase to the gel.

7. A method according to any one of Claims 1-6, **characterized** in that the matrix is formed from agarose or dextran.

8. A method according to Claim 1 **characterized** in that the ligands used have carbon chains which contain eight carbon atoms.

9. A method according to Claim 7 or 8, **characterized** in that the gel used is octyl agarose.

10. A method according to Claim 1, **characterized** in that the ligands contain one or more aromatic hydrocarbon groups.

11. A method according to Claim 10, **characterized** in that the gel used is a phenyl agarose gel.

## Patentansprüche

1. Verfahren zur Reinigung von Streptokinasen sowie Derivaten und Fragmenten derselben, dadurch gekennzeichnet, daß man eine Streptokinase in Lösung an ein Gel bindet, das eine Matrix mit lineare, verzweigte oder cyclische Kohlenwasserstoffgruppen mit mehr als vier Kohlenstoffatomen enthaltenden hydrophoben Liganden umfaßt, und man anschließend die an die Liganden gebundene Streptokinase von dem Gel eluiert.

2. Verfahren nach Anspruch 1, dadurch gekennzeichnet, daß man die Streptokinase an die Liganden bindet, indem man die Streptokinase in Lösung in einer Salzlösung mit einem Salzgehalt unterhalb des Sättigungsgehaltes mit dem Gel zusammenbringt.

3. Verfahren nach Anspruch 2, dadurch gekennzeichnet, daß das Salz ein Sulfat, Chlorid und/oder Phosphat eines Alkalimetalls oder von Ammonium oder mehrere dieser Salze ist.

4. Verfahren gemäß einem jeden der Ansprüche 1 bis 3, dadurch gekennzeichnet, daß die Streptokinase eluiert wird, indem man eine Salzlösung einer geringeren Ionenstärke als die Ionenstärke der zur Bindung der Streptokinase an das Gel verwendeten Lösung zuführt.

5. Verfahren gemäß einem jeden der Ansprüche 1 bis 3, dadurch gekennzeichnet, daß die Streptokinase eluiert wird, indem man eine Salzlösung zuführt, deren pH-Wert sich von dem pH-Wert der zur Bindung der Streptokinase an das Gel verwendeten Lösung unterscheidet.

6. Verfahren gemäß einem jeden der Ansprüche 1 bis 3, dadurch gekennzeichnet, daß die Streptokinase eluiert wird, indem man eine Lösung zuführt, die eine größere Hydrophilie als die zur Bindung der Streptokinase an das Gel verwendete Lösung aufweist.

7. Verfahren gemäß einem jeden der Ansprüche 1 bis 6, dadurch gekennzeichnet, daß die Matrix von Agarose oder Dextran gebildet ist.

8. Verfahren nach Anspruch 1, dadurch gekennzeichnet, daß die verwendeten Liganden Kohlenstoffketten aufweisen, die acht Kohlenstoffatome enthalten.

9. Verfahren nach Anspruch 7 oder 8, dadurch gekennzeichnet, daß das verwendete Gel Octylagarose ist.

10. Verfahren nach Anspruch 1, dadurch gekennzeichnet, daß die Liganden eine aromatische Kohlenwasserstoffgruppe oder mehrere enthalten.

11. Verfahren nach Anspruch 10, dadurch gekennzeichnet, daß das verwendete Gel ein Phenylagarosegel ist.

## Revendications

1. Procédé pour le nettoyage de streptokinases et de ses dérivés et fragments, caractérisé en ce que l'on fait lier la streptokinase en solution à un gel comprenant une matrice avec des ligands hydrophobes contenant des groupes hydrocarbures linéaires, ramifiés ou cycliques ayant plus de quatre atomes de carbone et en procédant ensuite à l'élution à partir de la streptokinase de gel qui s'est liée aux ligands.

2. Procédé selon la revendication 1, caractérisé en ce que la streptokinase est liée aux ligands par mise en contact de la streptokinase dans une solution saline ayant une teneur en sel au-dessous de la teneur de saturation avec le gel.

3. Procédé selon la revendication 2, caractérisé en ce que le sel est un ou plusieurs sulfates, chlorures et/ou phosphates de métaux alcalins ou d'ammonium.

4. Procédé selon l'une quelconque des revendications 1 à 3, caractérisé en ce que la streptokinase est éluée en alimentant une solution saline de résistance ionique inférieure à la résistance ionique de la solution utilisée pour lier la streptokinase au gel.

5. Procédé selon l'une quelconque des revendications 1 à 3, caractérisé en ce que la streptokinase est éluée en alimentant une solution dont le pH diffère du pH de la solution utilisée pour lier la streptokinase au gel.

6. Procédé selon l'une quelconque des revendications 1 à 3, caractérisé en ce que la streptokinase est éluée en amenant une solution qui possède une hydrophilie supérieure à la solution utilisée dans la liaison de la streptokinase au gel.

7. Procédé selon l'une quelconque des revendications 1 à 6, caractérisé en ce que la matrice est formée à partir d'agarose ou de dextrane.

8. Procédé selon la revendication 1, caractérisé en ce que les ligands utilisés possèdent des chaînes de carbone qui contiennent huit atomes de carbone.

9. Procédé selon la revendication 7 ou 8, caractérisé en ce que le gel utilisé est de l'octylagarose.

10. Procédé selon la revendication 1, caractérisé en ce que les ligands contiennent un ou plusieurs groupes d'hydrocarbures aromatiques.

11. Procédé selon la revendication 10, caractérisé en ce que le gel utilisé est un gel de phénylagarose.
